# EUROPEAN PATENT APPLICATION

(11) **EP 2 332 427 A1**
(43) Date of publication of application: **15.06.2011**
(21) Application number: 09178232.6
(22) Date of filing: 07.12.2009
(51) Int. Cl.: A23L 1/29, A23L 1/30, A61P 3/00, A61K 31/194, A61K 31/4439

(54) **low caloric fat replacers**

(71) Applicant: Nestec S.A., 1800 Vevey (CH)
(72) Inventor: Damak, Sami, 1066 Epalinges (CH); Godinot, Nicolas, 1010 Lausanne (CH); Le Coutre, Johannes, 1009 Pully (CH); Martin, Nathalie, Marguerite, Claire, 1005 Lausanne (CH)
(74) Representative: Marquardt, Ulf

(57) **Abstract**

The present invention relates in general to field of food and drinks. In particular, in relates to formulations that mimic the taste of fat but that are lower in calories. One embodiment of the present invention relates to the use of at least one non-fat agonist of GPR40 for imparting a fatty taste to a food product.

## Description

The present invention relates in general to field of food and drinks. In particular, in relates to formulations that mimic the taste of fat but that are lower in calories. One embodiment of the present invention relates to the use of at least one non-fat agonist of GPR40 for imparting a fatty taste to a food product.

A high intake of fatty compounds may be associated with an increased risk for the development of metabolic disorders, obesity and even cancer.

The consumption of saturated fats may be associated with increased blood cholesterol levels which may result in coronary heart disease, for example.

Because of this, the 1995 Dietary Guidelines (USDA and USDHHS, 1995) recommend limiting the total fat intake to no more than 30% of daily energy intake. Saturated fats should represent no more than 10% of the daily energy intake.

Consumed in appropriate amounts, fat has beneficial physiological properties. Additionally, it contributes to the essential sensory effects of a food product, such as flavour, mouthfeel, and aroma and increases the feeling of satiety achieved when consuming foods.

While consumer surveys indicate that 56% of adult Americans try to reduce fat intake, this desire is more likely to be successfully achieved, if the reduction of fat in a persons diet does not correspond to a reduced pleasure when consuming the food product.

Foods formulated with fat replacers are an enjoyable alternative compared to food compositions that are simply produced by replacing fat content with air or water.

Such fat replacers often are macromolecules that sometimes chemically resemble conventional fats and oils and which can replace the fat in foods by providing similar mouth feel and texture.

Typical fat replacers that are known today are shown in the following table 1:

**Table 1:**

| **Generic names, Brand Names** | **Compounds** |
|---|---|
| Sucrose polyesters, Olestra/Olean^{®} | Sucrose polyester of 6-8 fatty acids |
| Sucrose fatty acid esters | Sucrose with 1-3 fatty acids |
| Sorbestrin | Sorbitol, sorbitol anhydrides, fatty acids |
| Emulsifiers | Mono- and diacylglycerols, sodium stearoyl-2-lactylate, lecithin, sorbitan monostearate, propylene glycol mono- and diesters, diacetyl tartaric acid esters |
| Dialkyl dihexadecylmalonate | Fatty alcohol ester of malonic and alkyl malonic acids |
| Simplesse | A whey protein product |

Compounds that are chemically similar to fat compounds have the disadvantage that they usually have to be used in amounts that correspond to the amount of fat that is to be replaced in a weight ratio of about 1:1.

Non fat compounds that mimic the viscosity and texture of fat (for example Simplesse) often lack the taste of fat.

It would be desirable to have more potent compounds.

It would also be desirable to have available fat replacers that have a lower caloric value than the fat replacers of the prior art.

Even further, it would be desirable to have available fat replacers that are chemically not similar to fats. Chemically similar compounds to fat can often be treated only similarly to fats. Fat replacers with a different chemical nature than fats might offer new perspectives in the production and handling of fatty tasting compositions.

Even further it would be desirable to have available fat replacers chemically different from fat that mimic fat taste.

Hence it was the object of the present invention to provide alternative fat replacers. Advantageously such alternative fat replacers improve the state of the art by overcoming disadvantages of present fat replacers and/or by achieving at least one of the objects listed above.

The present inventors have achieved this object by the subject matter of the independent claim. The dependant claims develop the invention further.

The subject matter of the present invention provides improved fat replacers.

Preparations that mimic the sensation of fat and provide fewer calories are very useful to help control body weight. While the oral perception of fat has traditionally been considered to rely mainly on texture and olfaction, recent findings suggest that taste may also play a role in the detection of long chain fatty acids.

G-protein coupled receptors GPR40, originally found to be expressed in the pancreas, are activated by medium and long chain fatty acids.

The present inventors have now found that GPR40 is also expressed in the taste buds of humans and rodents.

While the activation of GPR40 by medium and long chain fatty acids in the pancreas can certainly not result in a taste sensation, the activation of GPR40 in taste buds may have an effect on taste.

The present inventors have further investigated this and found that GPR40 knockout mice show a diminished preference for medium and long chain fatty acids and diminished taste nerve responses to several fatty acids.

These results indicate that GPR40 expressed in taste buds is a fat taste receptor.

The natural agonists of GPR40 are medium and long chain fatty acids.

The present inventors have shown that by using examples of non-fatty acid agonists of GPR40, such as Roziglitazone and Medica 16, a fat taste can be produced from non-fat compounds.

Consequently, the fat replacers described in the present invention allow in particular to mimic the taste of fats, e.g., saturated fats.

These fat replacers can be combined with known fat replacers or combinations thereof, such as those mentioned in table 1, that primarily mimic the texture and olfaction of fat compounds to also impart a fat taste to food products and - consequently - to more accurately mimic the sensation of fat.

Using fatty tasting fat replacers of the present invention in food products will consequently lead to low-fat or non-fat products that are preferred by the consumer.

The subject matter of the present invention describes molecules that activate GPR40 (agonists) and taste fatty to humans. The invention hence relates to the use of at least one non-fatty acid agonist of GPR40 for imparting a fatty taste to a food product.

The use of the present invention may be a non-medical use.

While the non-fatty acid agonists of GPR40 may be used to support a fatty taste in all food products, they are preferably used as fat replacers.

Different from state of the art fat replacers that mimic texture and olfaction of fats, and that consequently usually have to be used in similar amounts as fat and have to be handled as fatty compounds, the fat replacers of the present invention produce a fat taste and may be used in different amounts by weight compared to the replaced fat. Due to the fact that the fat replacers of the present invention are chemically very different compounds compared to the replaced fats, these compounds can be treated differently than normal fats. In food production processes, this may be advantageous. It may be possible to apply higher temperatures, for example.

The fat replacer may be used alone or in combination with other fat replacers to replace fat in consumer goods.

It may be used to replace fat fully or partially.

Consequently, the non-fatty acid agonists of GPR40 may be used in a weight ratio of 100:1 to 1:1000000, preferably 50:1 to 1:5000, for example 10:1 to 1:100 compared to the natural lipid content of the food product.

Any amount of the non-fatty acid agonists of GPR40 will produce a fat taste in a dose dependant manner, as long as the GPR40 receptors are not fully saturated by the fat replacers of the present invention.

In the framework of the present invention, about 1 mmol of the non-fatty acid agonists of GPR40 may provide about the same fatty taste as 1 mmol of fatty acids.

Hence, the non-fatty acid agonists of GPR40 may be used in an amount (by mol) that about corresponds to the amount (by mol) of the replaced lipids.

Depending on the potency of the non-fatty acid agonists of GPR40 of the present invention, about 1 mmol of the non-fatty acid agonists of GPR40 may also provide much more fat taste than 1 mmol of fatty acids, e.g. 1 mmol of the non-fatty acid agonists of GPR40 may be used to provide the fat taste of at least than 5 mmol of fatty acids, for example at least 10 mmol of fatty acids.

Consequently, the non-fatty acid agonists of GPR40 may be used in a mol ratio of about 10:1 to 1:10, preferably of about 5:1 to 1:5, for example of about 2:1 to 1:2 compared to the replaced lipids.

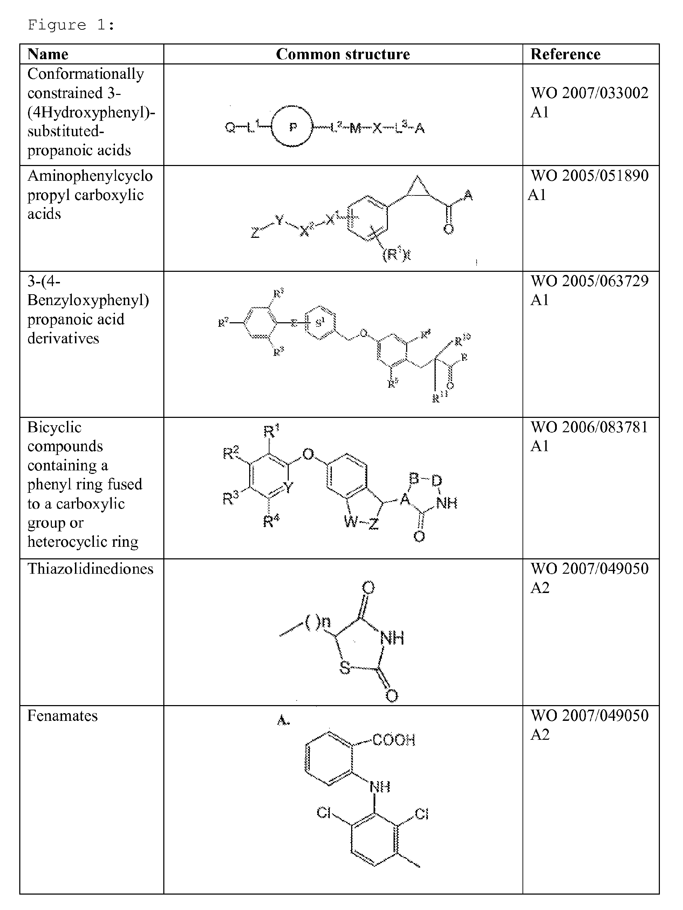

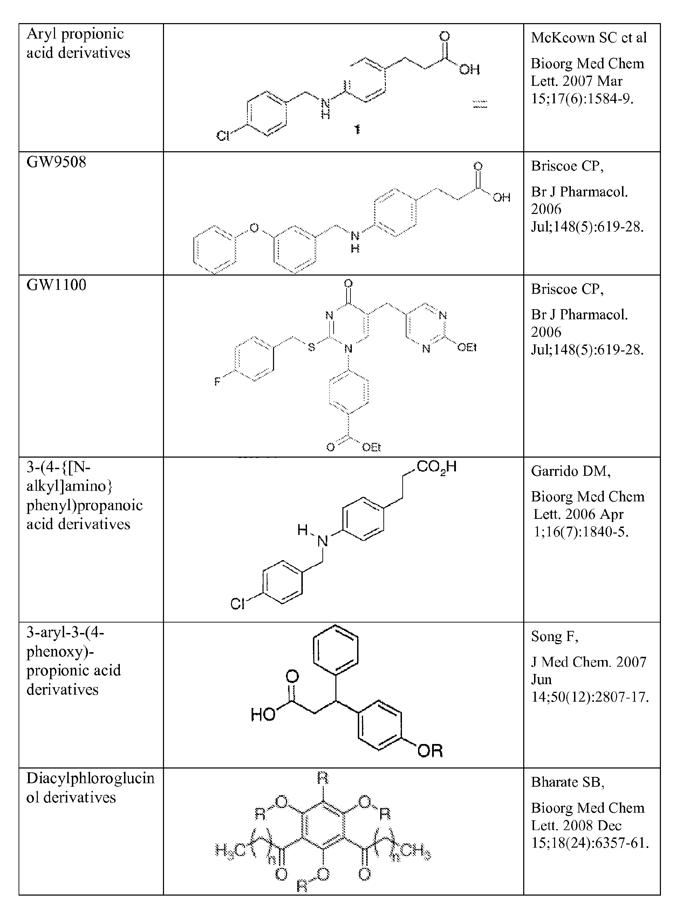

Non-fatty acid agonists of GPR40 are well known by those skilled in the art. Typical GPR40 agonists were described for example by Tan et al., Selective Small-Molecule Agonists of G protein-coupled Receptor 40 Promote Glucose-Dependent Insulin Secretion and Reduce Blood Glucose in Mice, Diabetes, 2008, published ahead of print online on May 13, 2008. Further well known non-fatty acid GPR40 agonists are presented in figure 1 along with the corresponding literature reference.

The content of these literature references is considered a part of this patent application.

Consequently, in the framework of the present invention the non-fatty acid agonists of GPR40 may be selected from the group consisting of Roziglitazone, Medica 16, conformationally constrained 3-(4 hydroxyphenyl)-substituted-propanoic acids, aminophenylcyclopropyl carboxylic acids, 3-(4-benzyloxyphenyl) propanoic acid derivatives, bicyclic compounds containing a phenyl ring fused to a carboxylic group or heterocyclic ring, thiazolidinediones (TZD), fenamates, aryl propionic acid derivatives, GW9508, GW1100, 3-(4-{[N-alkyl]amino} phenyl)propanoic acid derivatives, 3-aryl-3-(4-phenoxy)-propionic acid derivatives, diacylphloroglucinol derivatives, or combinations thereof.

The non-fatty acid agonists of GPR-40 may be used in any form to add a fatty taste to food products or drinks.

They may be provided as food supplement, for example.

The present invention also provides a food product.

"Food products" include drinks for the purpose of the present invention.

The food product enriched in at least one non-fatty acid agonist of GPR40 may but does not have to be a product with a reduced fat content.

The non-fatty acid agonists of GPR-40 may also be added to normal food products in order to strengthen the fatty taste of these products, since a fatty taste is normally perceived as pleasant. This will be even more appealing to consumers if the added pleasure does not come at the cost of added calories.

If the food product is a food product with a reduced fat content, at least 10%, preferably at least 25%, more preferably at least 50 %, even more preferably at least 75 %, most preferably at least 90 % of the natural fat content is replaced by non-fatty acid agonists of GPR40.

The food product may contain no lipid source. In such a case all lipids may have been replaced by fat replacers, for example completely or in part by the non-fatty acid agonists of GPR40 of the present invention.

The idea of adding a fat taste according to the present invention to food products may be applied to any food product.

The food product may for example be selected from the group consisting of nutritional formulas, ice creams, dairy products, creamers, pet food products, drinks, nutraceuticals, food additives, confectionary, chocolate based products, seasoning products, mayonnaise, soups, frozen meals, cakes, and deserts.

Food products comprising the non-fatty acid agonists of GPR40 have a reduced caloric content are more enjoyable that other low-fat products, since the fat taste is still present in these products.

Hence, these products will be more regularly consumed, which has a positive health effect for the consumer.

Hence, the food products comprising and/or enriched in the non-fatty acid agonists of GPR40 of the present invention may be for use in weight management.

The food products described in the present invention may be also for use in the treatment or prevention of overweightness and/or obesity.

"Overweight" is defined for an adult human as having a BMI between 25 and 30.

"Body mass index" or "BMI" means the ratio of weight in kg divided by the height in metres, squared.

"Obesity" is a condition in which the natural energy reserve, stored in the fatty tissue of animals, in particular humans and other mammals, is increased to a point where it is associated with certain health conditions or increased mortality. "Obese" is defined for an adult human as having a BMI greater than 30.

During the past decades, the prevalence of obesity has increased worldwide to epidemic proportion. Approximately 1 billion people worldwide are overweight or obese, conditions that increase mortality, morbidity and economical costs. Obesity develops when energy intake is greater than energy expenditure, the excess energy being stored mainly as fat in adipose tissue. Body weight loss and prevention of weight gain can be achieved by reducing energy intake or bioavailability, increasing energy expenditure and /or reducing storage as fat. Obesity represents a serious threat to health because it is associated with an array of chronic diseases, including diabetes, atherosclerosis, degenerative disorders, airway diseases and some cancers.

Since establishing and maintaining a proper body weight and - in particular - an acceptable weight percentage of fat in the body is a key step to treat or prevent metabolic disorders, the food product in accordance the present invention may be for use in the treatment or prevention of metabolic disorders.

Typical metabolic disorders include but are not limited to diabetes, hypertension, liver cirrhosis, metabolic syndrome, and/or cardiovascular diseases.

The food product of the present invention can hence make a significant contribution to the well-being of today's population, in particular in well developed countries.

The present inventors have shown that an agonistic modulation of the GPR-40 receptor will result in the perception of a fatty taste.

Consequently, the agonistic modulation of the GPR-40 receptor may be used to identify further compounds with a fatty taste.

This can be done by means of a bioassay, for example. Typical bioassays that may be used for this purpose are well known to those skilled in the art and include for example, cell based calcium or fluorescence imaging.

The subject matter of the present invention extends hence to the use of the GPR40 receptor to identify compounds with a fatty taste.

For example, the present invention relates to the use of a GPR40-based bioassay to identify non-fatty acid compounds with a fatty taste.

Those skilled in the art will understand that they can freely combine all features of the present invention described herein, without departing from the scope of the invention as disclosed. In particular, features described for the use of the present invention may be applied to the food product of the present invention and vice versa.

Further advantages and features of the present invention are apparent from the following Examples and Figures.

Figure 1 shows typical GPR40 agonists known in the art.

Figure 2 shows the proportion of correct choices obtained in 2-Alternative forced choice tests performed by 40 subjects. A correct choice is defined as choosing as fattier the sample containing the test ingredients. Medica 16 and rosiglitazone were assessed independently at three concentration, 10µM, 50µM and 100µM. The two higher concentrations of both compound were significantly chosen as fattier than control solution.

### Example:

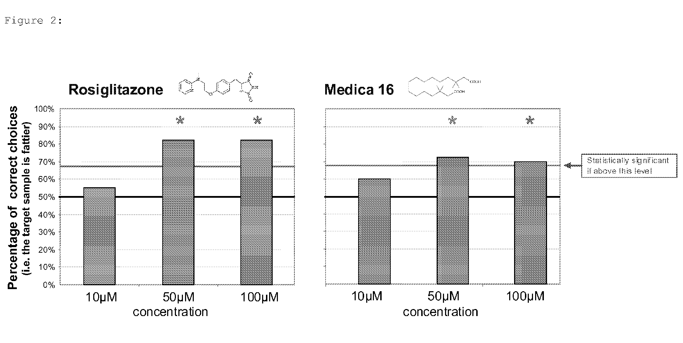

Forty subjects were given two solutions one containing the agonist and the other water, and asked which one tastes fattier. For both Roziglitazone and Medica 16 at either 50 µM or 100 µM, more subjects found the solution with agonists to taste fattier (p<0.05, Figure 2).

## Claims

1. Use of at least one non-fatty acid agonist of GPR40 for imparting a fatty taste to a food product.

2. Use according to claim 1, wherein the non-fatty acid agonists of GPR40 are used as fat replacers.

3. Use according to one of the preceding claims, wherein the non-fatty acid agonists of GPR40 are used in a weight ratio of 100:1 to 1:1000000, preferably 50:1 to 1:5000, for example 10:1 to 1:100 compared to the natural lipid content of the food product.

4. Use according to one of the preceding claims, wherein about 1 mmol the non-fatty acid agonists of GPR40 provides the same fatty taste as 1 mmol of fat. ?

5. Use according to one of the preceding claims, wherein the non-fatty acid agonists of GPR40 are selected from the group consisting of Roziglitazone, Medica 16, conformationally constrained 3-(4 hydroxyphenyl)-substituted-propanoic acids, aminophenylcyclopropyl carboxylic acids, 3-(4-benzyloxyphenyl) propanoic acid derivatives, bicyclic compounds containing a phenyl ring fused to a carboxylic group or heterocyclic ring, thiazolidinediones (T2D), fenamates, aryl propionic acid derivatives, GW9508, GW1100, 3-(4-{[N-alkyl]amino} phenyl)propanoic acid derivatives, 3-aryl-3-(4-phenoxy)-propionic acid derivatives, diacylphloroglucinol derivatives, or combinations thereof.

6. Food product enriched in at least one non-fatty acid agonist of GPR40.

7. Food product in accordance with claim 6, which is a product with a reduced fat content.

8. Food product in accordance with one of claims 6-7, wherein at least 10%, preferably at least 25%, more preferably at least 50 %, even more preferably at least 75 %, most preferably at least 90 % of the natural fat content are replaced by non-fatty acid agonists of GPR40.

9. Food product in accordance with one of claims 6-8, wherein the food product contains no lipid source.

10. Food product in accordance with one of claims 6-9, wherein the food product is selected from the group consisting of nutritional formulas, ice creams, dairy products, creamers, pet food products, drinks, nutraceuticals, food additives, confectionary, chocolate based products, seasoning products, mayonnaise, soups, frozen meals, cakes, deserts.

11. Food product in accordance with one of claims 6-10 for use in weight management.

12. Food product in accordance with one of claims 6-10 for use in the treatment or prevention of overweightness and/or obesity.

13. Food product in accordance with one of claims 6-10 for use in the treatment or prevention of metabolic disorders.

14. Use of a GPR40-based bioassay to identify non-fatty acid compounds with a fatty taste.
